# DEMANDE DE BREVET EUROPEEN

(11) **EP 4 374 844 A1**
(43) Date de publication de la demande: **29.05.2024**
(21) Numéro de dépôt: 22209568.9
(22) Date de dépôt: 25.11.2022
(51) Int. Cl.: A61K 8/02, A61K 8/19, A61K 8/20, A61K 8/27, A61Q 1/00, A61Q 1/02

(54) **COMPOSITION DE COULEUR BLANCHE ET SON UTILISATION DANS DES FORMULATIONS DIVERSES EN PARTICULIER COSMETIQUES**

(71) Demandeur: Chanel Parfums Beauté, 92200 Neuilly-sur-Seine (FR); Olikrom, 33600 Pessac (FR)
(72) Inventeur: GUARILLOFF, Philippe, 93500 PANTIN (FR); LETARD, Jean-François, 33610 CANEJAN (FR)
(74) Mandataire: Aquinov

(57) **Abrégé**

La présente invention concerne une composition visant à remplacer le TiO2, son procédé de fabrication et ses utilisations. En particulier la présente invention a pour objet une composition de couleur blanche, insoluble dans l'eau, présentant une clarté définie par un paramètre L^{∗} supérieur à 90, une opacité supérieure à 80%, comprenant le mélange d'oxychlorure de Bismuth, de nitrure de bore et d'au moins un autre composé inorganique. Ladite composition est capable de se substituer au dioxyde de Titane adaptée à des différentes utilisations notamment cosmétiques. L'invention concerne également son procédé de préparation ainsi que ses utilisations.

## Description

### Domaine technique

La présente invention se rapporte au domaine technique des pigments. L'invention a en particulier pour objet une composition particulière de couleur blanche capable de se substituer au dioxyde de Titane (TiO2), notamment en cosmétiques. L'invention concerne également son procédé de préparation ainsi que ses utilisations.

### Art antérieur

Les pigments sont fondamentaux dans la composition de certains produits pour donner de la couleur. Ils sont notamment très utilisés dans les produits cosmétiques car ils donnent de la couleur et de l'opacité aux produits finis. Dans les produits de maquillage, les pigments sont essentiels cars ils participent au rendu sur la peau ou sur les ongles. Les pigments sont également présents dans les produits de coloration des cheveux ou encore dans les produits de soin pour donner une coloration spécifique sur la peau.

Les pigments sont des molécules solides et insolubles. Ils peuvent être d'origine animale, végétale, ou synthétique, et peuvent être de nature inorganique, organique.

De nos jours, les pigments les plus utilisés en industrie sont les pigments d'origine inorganique. En effet, leur résistance à la lumière et leur faible cout en font des matériaux très recherchés. Ils sont constitués de minéraux broyés secs, généralement des métaux ou des sels métalliques. En raison de leur composition, les pigments inorganiques sont généralement plus opaques et plus insolubles que les pigments organiques.

Parmi les pigments minéraux très utilisés, on peut citer en particulier le TiO2. Il s'agit d'un pigment inorganique utilisé sous forme de poudre dans de nombreuses applications pour sa couleur blanche incomparable et ses nombreuses autres propriétés telles que l'absorption des rayons ultraviolets, ses propriétés opacifiantes, sa résistance aux produits chimiques, sa stabilité thermique et son potentiel photocatalyseur.

Grace à ses propriétés remarquables, le TiO2 entre dans la formulation d'une grande variété de produits finis tels que des peintures, des encres, des médicaments, des dentifrices, des produits de construction (bâtiment et travaux publics) et des formulations de produits cosmétiques.

Avec l'évolution des réglementations, de plus en plus d'études portent sur l'innocuité des pigments sur l'homme et l'environnement. Certaines études récentes ont pu montrer le potentiel cancérigène du TiO2 par inhalation.

Dans le domaine de la cosmétique, afin d'éviter son inhalation, le TiO2 n'est plus employé sous forme de poudre de particules de petite taille dans certaines formulations telles que les sprays.

Bien que son utilisation dans les produits pour application topique ne soit pas interdite, les industriels, notamment en cosmétique sont toujours à la recherche de nouveaux composés pouvant proposer une alternative à l'utilisation omniprésente du TiO2 qui soit adapté à une utilisation sur la peau et les phanères.

### Résumé de l'invention

Pour répondre à ce besoin, l'invention propose une nouvelle composition capable de constituer une alternative au TiO2 adaptée en particulier à une utilisation en cosmétique.

En particulier, l'invention a pour objet une composition de couleur blanche, insoluble dans l'eau, présentant une clarté définie par un paramètre L^{∗} supérieur à 90, une opacité supérieure à 80%, comprenant le mélange d'oxychlorure de bismuth, de nitrure de bore et d'au moins un autre composé inorganique.

Préférentiellement la composition selon l'invention est sous forme de poudre.

Elle présente des propriétés équivalentes à celles du TiO2 à savoir une clarté définie par un paramètre L^{∗} supérieur à 90 et une opacité supérieure à 80%.

L'oxychlorure de bismuth est un pigment inorganique insoluble dans l'eau pouvant être obtenu à partir de cuivre, d'étain ou de plomb. Il est utilisé dans de nombreux domaines, notamment en cosmétique dans des ombres à paupières, du rouge à lèvres et des vernis à ongles. L'oxychlorure de bismuth présente une clarté L^{∗} de 85 et une opacité de 85%. Ainsi, l'utilisation de l'oxychlorure de bismuth seul est insuffisante pour constituer une alternative au TiO2, en particulier en cosmétique.

Le nitrure de bore est un composé chimique de couleur blanche utilisé dans de nombreux domaines, notamment en cosmétique. Par ses propriétés structurelles, le nitrure de bore peut prendre différentes conformations, notamment en lamelles, en nid d'abeille. Cette conformation dite hexagonal lui permet d'apporter un toucher doux et soyeux améliorant l'étalement de la formulation le comprenant, notamment sur la peau. Le nitrure de bore présente une clarté L^{∗}de 90 et une opacité de 86%. Ainsi, l'utilisation du nitrure de bore seul est insuffisante pour constituer une alternative au TiO2, en particulier en cosmétique.

Avantageusement, la combinaison des composés selon l'invention permet d'engendrer un effet synergique sur la clarté et/ou l'opacité de la composition qui sont supérieures à la clarté et/ou l'opacité de chacun des composés constituant la composition pris individuellement.

La composition selon l'invention présente une clarté lui permettant d'être adaptée à son utilisation en particulier dans les formulations cosmétiques, les médicaments, les encres et peintures comme substitut au TiO2.

Selon un mode de réalisation particulièrement adapté, la composition selon l'invention comprend le mélange d'au moins 4 composés inorganiques.

Préférentiellement, la composition selon l'invention comprend également, en plus du mélange d'oxychlorure de bismuth, de nitrure de bore au moins un autre composé inorganique choisi parmi oxyde de fer, oxyde de zinc, oxyde de cérium, carbonate de calcium, phosphate tricalcique, sulfate de calcium, stéarate de zinc, carbonate de magnésium, sulfate de zinc, stéarate de magnésium, sulfate de baryum, sulfure de zinc, poudre d'aluminium, argile calcinée, phosphate tricalcique, kaolin et leur combinaison.

Selon un mode de réalisation préféré de l'invention, la composition présente une prise d'huile inférieure à 55%.

Une telle composition est particulièrement utile pour apporter une alternative à l'utilisation du TiO2 dans différentes formulations et applications, en particulier en cosmétique.

Selon un autre aspect, l'invention concerne un procédé de fabrication d'une composition selon l'invention comprenant notamment une étape de sélection des composés constituant la composition selon l'invention, un mélange desdits constituants et une récupération de la composition selon l'invention.

Enfin, l'invention vise aussi l'utilisation de la composition selon l'invention dans des encres, des peintures, des plastiques et des formulations de produits cosmétiques.

D'autres caractéristiques et avantages ressortiront de la description détaillée de l'invention et des exemples uniquement illustratifs et nullement limitatifs de la portée de l'invention.

### Description détaillée de l'invention

### Définitions

Par « clarté » au sens de l'invention, on entend une indication sur la blancheur de la couleur étudiée. Plus la couleur est sombre, plus la clarté est faible. Pour mesurer la clarté, il est nécessaire de déposer la composition selon l'invention sur des cartes de contraste de fond noir en formant un film d'épaisseur uniforme. La mesure est obtenue à l'aide d'un spectrocolorimètre équipé d'une sphère d'intégration et d'un illuminant D65. Plus le paramètre L^{∗} se rapproche de 100, plus la composition est blanche.

Par « opacité » au sens de l'invention, on entend la capacité d'une composition à occulter une surface couverte par une quantité donnée. La mesure de l'opacité s'effectue en mesurant son pouvoir couvrant. On peut mesurer l'opacité en calculant le rapport de contraste sur fond blanc et noir. L'application de la composition peut s'effectuer à l'aide d'un filmographe à une épaisseur connue. Après un temps de séchage, un spectrophotomètre mesure une valeur de réflectance pour le recouvrement sur fond blanc (Yw) et sur fond noir (Yb). On mesure ainsi l'opacité d'une composition en calculant le rapport de contraste Yb/Yw que l'on exprime en pourcentage.

Par « réflectance » au sens de l'invention, on entend la mesure de la proportion de lumière réfléchie sur une surface.

Par « prise d'huile » au sens de l'invention, on entend la quantité d'huile de lin (en grammes) absorbée par 100g de composition selon l'invention pour obtenir une pâte ferme et homogène. Plus particulièrement la mesure de la prise d'huile selon l'invention consiste à malaxer la composition selon l'invention avec la quantité minimale d'huile de lin pour obtenir une masse plastique (poudre + huile) qui se détache aisément du support (spatule et/ou plaque de verre) sur laquelle est effectuée le malaxage. Plus cette valeur est grande, plus la composition absorbe l'huile.

Par « polymère filmogène » au sens de l'invention, on entend un polymère apte à former à lui seul ou en présence d'un agent auxiliaire de filmification, un film continu et adhérent sur un support, notamment sur les matières kératiniques.

Par « cire » au sens de l'invention, on entend une matière grasse à changement réversible de phase liquide/solide, ayant une température de fusion supérieure à 55°C et généralement inférieure à 110°C, qui est liquide dans les conditions de préparation de la formulation selon l'invention et qui présente une organisation cristalline anisotrope à l'état solide. La cire peut être polaire ou apolaire Par « cire polaire » au sens de l'invention, on entend une cire comprenant au moins un hétéroatome tel que l'oxygène, l'azote, le silicium ou le phosphore.

Par « cire apolaire », au sens de l'invention, on entend une cire hydrocarbonée et/ou une cire siliconée.

Par « charge » au sens de l'invention, on entend toute particule de forme quelconque (notamment sphérique ou lamellaire), minérale ou organique, insoluble dans la formulation.

### Composition

La présente invention a donc pour objet une composition de couleur blanche, insoluble dans l'eau, présentant une clarté définie par un paramètre L^{∗} supérieur à 90, une opacité supérieure à 80% et comprenant le mélange d'oxychlorure de bismuth, de nitrure de bore et d'au moins un autre composé inorganique.

Avantageusement, la composition selon l'invention présente une clarté et une opacité équivalente à celle du TiO2.

Selon un mode de réalisation particulièrement préféré, la composition selon l'invention ne comprend pas de TiO2.

La clarté de la composition selon l'invention est évaluée en mesurant la coordonnée chromatique L^{∗} issue de l'espace chromatique CIELAB, généralement nommé CIE 1976, à l'aide d'un spectrocolorimètre à sphère d'intégration sous éclairage D65.

De façon préférée, la composition selon l'invention présente une clarté supérieure à 91, notamment supérieure à 92, préférentiellement supérieure à 95.

Avantageusement, plus la clarté de la composition selon l'invention est élevée plus la composition est blanche.

L'opacité de la composition selon l'invention est évaluée en mesurant son pouvoir couvrant à l'aide d'un spectrophotomètre mesurant une valeur de réflectance de la composition appliquée sur fond blanc (Yw) et sur fond noir (Yb).

De façon préférée, la composition selon l'invention présente une opacité supérieure à 82%, notamment supérieure à 85%, préférentiellement supérieure à 90%, encore plus préférentiellement supérieure à 92%.

Selon un mode de réalisation préféré, la composition selon l'invention présente une clarté L^{∗} supérieure à 91 et/ou une opacité supérieure à 90%. Selon une variante particulièrement préférée, la composition selon l'invention présente une clarté L^{∗} supérieure ou égale à 92, et une opacité supérieure ou égale à 91%.

Préférentiellement, la composition selon l'invention présente une clarté supérieure et/ou une opacité supérieure à celle de chacun des composés constituant la composition.

Avantageusement, les composés sélectionnés présentent un effet synergique sur le paramètre L^{∗} mesuré sur fond noir et sur l'opacité.

Lorsque la composition est utilisée dans des encres ou peinture, la composition selon l'invention conserve avantageusement ses caractéristiques, notamment sa clarté et son opacité, même après séchage. Après application, la composition selon l'invention peut former un film sec conservant son organisation structurelle, notamment son opacité et sa clarté.

La composition selon l'invention comprend un mélange d'au moins 3 composés inorganiques, préférentiellement au moins 4, encore plus préférentiellement au moins 5.

De façon préférée, la composition selon l'invention comprend également, en plus du mélange d'oxychlorure de bismuth et de nitrure de bore, au moins un autre composé inorganique choisi parmi oxyde de fer, oxyde de zinc, oxyde de cérium, carbonate de calcium, phosphate tricalcique, sulfate de calcium, stéarate de zinc, carbonate de magnésium, sulfate de zinc, stéarate de magnésium, sulfate de baryum, sulfure de zinc, poudre d'aluminium, argile calcinée, kaolin et leur combinaison.

Selon un mode de réalisation particulièrement préféré, la composition selon l'invention comprend également, en plus du mélange d'oxychlorure de bismuth et de nitrure de bore, au moins un autre composé inorganique choisi parmi le stéarate de zinc, le sulfate de zinc, l'oxyde de zinc et le sulfure de zinc.

Selon un mode de réalisation, la composition selon l'invention comprend le mélange d'oxychlorure de bismuth et de nitrure de bore représentant entre 10 et 95% en poids, préférentiellement entre 15 et 90% en poids par rapport au poids total de la composition.

Selon une variante, la composition selon l'invention comprend le mélange d'oxychlorure de bismuth et de nitrure de bore représentant entre 50 et 95%, préférentiellement entre 65 et 95%, plus préférentiellement entre 80 et 95% en poids par rapport au poids total de la composition.

Selon une autre variante, la composition selon l'invention comprend le mélange d'oxychlorure de bismuth et de nitrure de bore représentant entre 10 et 30%, préférentiellement entre 10 et 25%, plus préférentiellement entre 10 et 20% en poids par rapport au poids total de la composition.

Selon un mode de réalisation, la composition comprend un mélange d'oxychlorure de bismuth et de nitrure de bore présentant un ratio compris entre 0,1/1 et 0,7/1, préférentiellement un ratio compris entre 0,11/1 et 0,65/1.

Selon un autre mode de réalisation préféré, la composition selon l'invention présente une prise d'huile inférieure à 55%, préférentiellement inférieure à 40%, notamment comprise entre 25 et 55%, encore plus préférentiellement comprise entre 35 et 45%.

Avantageusement, la prise d'huile de la composition selon l'invention permet de faciliter son intégration dans des formulations de produits cosmétiques.

Préférentiellement la composition selon l'invention est sous forme de poudre.

La composition selon l'invention est ainsi particulièrement adaptée pour remplacer le TiO2 dans des formulations de produits cosmétiques.

Selon un autre aspect, la composition selon l'invention est susceptible d'être obtenue par un procédé de fabrication comprenant les étapes suivantes :
Sélection de chaque composé constituant la composition selon l'invention : oxychlorure de bismuth, nitrure de bore et au moins un autre composé inorganique
Mélange des composés sélectionnés à l'étape a) par ajout successif

Récupération d'une composition de couleur blanche, présentant une clarté définie par un paramètre L^{∗} supérieur à 90, une opacité supérieure à 80%, et optionnellement une prise en huile inférieure à 55%.

L'invention concerne également une formulation comprenant la composition selon l'invention.

Selon un mode de réalisation, la formulation selon l'invention comprend entre 0,1 et 20% en poids de composition selon l'invention, préférentiellement entre 0,5 et 10% et encore plus préférentiellement entre 1 et 8%, par rapport au poids total de la formulation.

La formulation selon l'invention peut comprendre un milieu cosmétiquement acceptable, c'est-à-dire susceptible d'être appliqué sur la peau, les lèvres ou les cils sans générer d'inconforts pour l'utilisateur.

La formulation selon l'invention peut se présenter notamment sous la forme d'une formulation de mascara, d'une formulation de rouge à lèvres ou brillant à lèvres, sous la forme d'un fard à paupières et/ou à joues ou encore sous la forme d'une formulation de fond de teint ou de produit de soin pour le visage ou pour le corps.

De façon préférée, la formulation selon l'invention peut se présenter sous toutes les formes adaptées au domaine de la cosmétique, notamment sous la forme d'une poudre, d'une émulsion, d'une microémulsion, d'une nanoémulsion, d'une suspension, d'une solution, d'une lotion, d'une crème, d'un gel aqueux ou hydroalcoolique, d'une mousse, d'un sérum, d'une solution ou d'une dispersion pour aérosol, ou d'une dispersion de vésicules lipidiques, ou de gouttelettes obtenues par coacervation ou pas microfluidique.

Selon un mode de réalisation, la formulation selon l'invention se présente sous forme de formulation anhydre, c'est-à-dire renfermant moins de 5% en poids d'eau, préférentiellement moins de 1% en poids d'eau.

Selon un autre mode de réalisation, la formulation selon l'invention se présente sous forme d'émulsion eau-dans-huile ou huile-dans-eau.

La formulation selon l'invention, peut également comprendre en plus de la composition selon l'invention au moins un additif choisi parmi un agent émollient, un agent humectant, un agent gélifiant et/ou épaississant, un agent filmogène, un agent tensioactif, une huile, un solvant organique non volatile, une charge, une cire, un agent actif, un colorant, un conservateur, un agent antioxydant, une poudre organique ou inorganique, un filtre solaire et un parfum.

Lorsque la formulation selon l'invention comprend au moins un agent humectant, au moins un agent humectant est choisi parmi :
- les polyols tels que laglycérine, la diglycérine, le propylène glycol, le caprylyl glycol, le pentylène glycol ou l'hexanediol) ;
- les sucres ;
- les glycosaminoglycanes tels que l'acide hyaluronique et ses sels et esters ; et
- les polyquaterniums tel que le lipidure PMB.

De façon préférée, la formulation selon l'invention comprend en plus de la composition selon l'invention, au moins un agent humectant ayant une teneur comprise entre 0 et 30%, de préférence entre 0,005 et 10% en poids total de la formulation.

Lorsque la formulation selon l'invention comprend au moins un agent émollient, au moins un agent émollient est choisi parmi :
- les esters tels que les esters de jojoba, les esters d'acides gras et d'alcool gras, préférentiellement choisi parmi octyldodecyl myristate, triethylhexanoin, Dicaprylyl carbonate et Isostearyl isostearate, caprylic/capric triglyceride ;
- les beurres tels que les beurres de karité préférentiellement butyrospernum parkii butter extract, shea butter ethyl esters, commercialisés sous les noms de LIPEX SHEASOFT, LIPEX SHEA-U, LIPEX SHEA, LIPEX SHEALIGHT, LIPEX SHEA TRIS ou les beurres de moringa préférentiellement moringa oil/hydrogenated moringa oil esters) ;
- les cires préférentiellement choisi parmi acacia decurrens flower wax, helianthus annuus cera seed seed wax, C10-18 triglycérides ;
- les huiles végétales ;
- le phytosqualane
- les alcanes préférentiellement choisi parmi undécane et tridécane.

De façon préférée, la formulation selon l'invention comprend en plus de la composition selon l'invention, au moins un agent émollient ayant une teneur comprise entre 0,1 et 30%, de préférence entre 0,5 et 10% en poids total de la formulation.

Lorsque la formulation selon l'invention comprend au moins un agent gélifiant et/ou épaississant, au moins un agent gélifiant et/ou épaississant est choisi parmi :
- les dérivés cellulosiques préférentiellement choisi parmi les gommes d'origine végétale tels que le guar, caroube, alginates, carraghenanes, pectines, les gommes d'origine microbienne tels que le xanthane,
- les argiles tels que la laponite
- les homo- et copolymères réticulés ou non, hydrophiles ou amphiphiles
- l'acide acryloylméthylpropane sulfonique (AMPS) et/ou d'acrylamide et/ou d'acide acrylique et/ou de sels ou d'esters d'acide acrylique (commercialisés sous les noms ARISTOFLEX AVC, Aristoflex AVS, Aristoflex HMB, SIMULGEL NS, Simulgel EG, Simulgel 600, Simulgel 800, Pemulen, carbopol, Sepiplus 400, Seppimax zen, Sepiplus S, COSMEDIA SP).

De façon préférée, la formulation selon l'invention comprend en plus de la composition selon l'invention, au moins un agent gélifiant et/ou épaississant ayant une teneur comprise entre 0,1 et 10% en poids total de la formulation.

Lorsque la formulation selon l'invention comprend au moins un agent tensionactif, au moins un agent tensioactif est choisi parmi :
- les tensioactifs anioniques tels que les isethionates, les taurates, les sarcosinates, les glycinates, les glutamates, les phosphates (C20-22 alkyl phosphate commercialisé sous le nom SENSANOV WR) ;
- les tensioactifs amphotères tel que les dérivés de la bétaïne, les amphoacétates ;
- les tensioactifs non ioniques tel que les dérivés de polyglycérol, les dérivés de sucre tels que les dérivés de glucosides ou de xylosides commercialisés sous le nom MONTANOV 68, MONTANOV 202, Montanov 82, MONTANOV L, EASYNOV), les lécithines.

De façon préférée, la formulation selon l'invention comprend en plus de la composition selon l'invention, au moins un agent tensioactif ayant une teneur comprise entre 0,1 et 15%, de préférence entre 0,5 et 10% en poids total de la formulation.

Lorsque la formulation selon l'invention comprend au moins un polymère filmogène, au moins un polymère filmogène est choisi parmi :
- les polymères synthétiques tels que les polymères de type radicalaire ou de type polycondensat,
- les polymères d'origine naturelle et leurs mélanges, préférentiellement les polymères acryliques, les polyhydroxyalcanoate (PHA), les polyuréthanes, les polyesters, les polyamides, les polyurées et les polymères cellulosiques tels que la nitrocellulose.

De façon préférée, la formulation selon l'invention comprend en plus de la composition selon l'invention, au moins un polymère filmogène ayant une teneur comprise entre 0,5 et 20% en poids total de la formulation.

Avantageusement, le polymère filmogène permet d'améliorer la tenue et les propriétés de non-transfert de la formulation selon l'invention, notamment aux formulations de maquillage.

Selon un mode de réalisation de l'invention, la formulation comprend en plus de la composition selon l'invention, au moins une huile ou un solvant organique non volatile choisie parmi les huiles hydrocarbonées et/ou siliconées non volatiles.

Lorsque la composition comprend au moins une huile ou un solvant organique non volatile, il/elle présente une teneur comprise entre 0,01 et 30% en poids, préférentiellement entre 0,1 et 25%, notamment entre 0,1 et 20% par rapport au poids total de la formulation.

De façon préférée, la formulation selon l'invention comprend en plus de la composition selon l'invention, au moins une huile ou un solvant organique non volatile ayant une teneur entre 0,01 et 30% en poids, préférentiellement entre 0,1 et 25%, notamment entre 0,1 et 20% par rapport au poids total de la formulation.

Selon un autre mode de réalisation, la formulation selon l'invention comprend en plus de la composition selon l'invention, au moins une cire ayant une teneur comprise entre 0,5 et 20%, de préférence entre 1 et 10% en poids total de la formulation.

Lorsque la formulation selon l'invention comprend au moins une charge, au moins une charge est choisie parmi le talc, le mica, la silice, le kaolin, le nitrure de bore, l'amidon, l'amidon modifié par l'anhydride octénylsuccinique, les polyamides, les résines de silicone, les poudres d'élastomères de silicone et les poudres de polymères acryliques telles que le poly-méthacrylate de méthyle. Les charges utilisées dans la formulation selon l'invention peuvent notamment être constituées de plusieurs couches de nature chimique et/ou de forme physique différentes et notamment se présenter sous forme de lamelles enrobées de charges sphériques. Elles peuvent être modifiées à l'aide de différents traitements de surface.

Selon un mode de réalisation, la formulation selon l'invention comprend en plus de la composition selon l'invention, au moins une matière colorante choisie parmi un pigment ou une nacre.

Lorsque la formulation selon l'invention comprend au moins une matière colorante comprenant un pigment, le pigment peut être blancs ou colorés, minéraux et/ou organiques, enrobés ou non.

Ledit pigment est préférentiellement choisi parmi les oxydes de zirconium, de zinc ou de cérium, ainsi que les oxydes de fer ou de chrome, le violet de manganèse, le bleu outremer, l'hydrate de chrome et le bleu ferrique, le noir de carbone, les pigments de type D & C, et les laques à base de carmin de cochenille, de baryum, strontium, calcium, aluminium.

Les nacres peuvent être préférentiellement choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

D'autres additifs habituellement utilisés en cosmétique peuvent également être présents dans la formulation selon l'invention, notamment des conservateurs, des agents antioxydants ou des parfums bien connus dans le domaine technique.

L'homme du métier est en mesure de choisir, parmi l'ensemble de ces éventuels additifs, aussi bien la nature que la quantité de ceux qui seront ajoutés à la composition, de telle sorte que celle-ci conserve l'ensemble de ses propriétés.

### Procédé

Aussi, selon un autre aspect, l'invention a pour objet un procédé de fabrication d'une composition selon l'invention comprenant au moins la mise en œuvre des étapes suivantes :
Sélection de chaque composé constituant la composition selon l'invention : oxychlorure de bismuth,
nitrure de bore et au moins un autre composé inorganique
Mélange des composés sélectionnés à l'étape a) par ajout successif ; et

Récupération d'une composition de couleur blanche, présentant une clarté définie par un paramètre L^{∗} supérieur à 90, une opacité supérieure à 80%, et optionnellement une prise en huile inférieure à 55%.

Les composés de l'étape a) sont des composés inorganiques différents :
- au moins l'oxychlorure de bismuth et le nitrure de bore ; et
- au moins un autre composé inorganique choisi parmi : oxyde de fer, oxyde de zinc, oxyde de cérium, carbonate de calcium, phosphate tricalcique, sulfate de calcium, stéarate de zinc, carbonate de magnésium, sulfate de zinc, stéarate de magnésium, sulfate de baryum, sulfure de zinc, poudre d'aluminium, argile calcinée, phosphate tricalcique, kaolin et leur combinaison.

Le mélange de l'étape b) est réalisé par convection, diffusion ou dispersion mécanique, préférentiellement le mélange de l'étape b) est effectué à l'aide d'un mélangeur à poudre.

Avantageusement, le mélangeur à poudre permet de mélanger la composition selon l'invention sans provoquer d'écrasement de la composition sur les parois.

De façon préférée, le mélange de l'étape b) est réalisé dans un mélangeur à poudre à une vitesse comprise entre 1 et 20 tr/min, préférentiellement entre 1 et 10 tr/min, encore plus préférentiellement à 10 tr/min.

Avantageusement, la vitesse du mélange de l'étape b) permet d'obtenir un mélange homogène.

Préférentiellement, le mélange de l'étape b) est effectué pendant une durée comprise entre 1 à 10 heures préférentiellement de 1 à 5 heures, encore plus préférentiellement 2 heures.

Selon un mode de réalisation particulièrement préféré, le mélange de l'étape b) est réalisé à l'aide d'un mélangeur à poudre à une vitesse de 5 tr/min pendant 2 heures.

Avantageusement, le mélange de l'étape b) permet d'obtenir une composition homogène.

Ainsi, le procédé selon l'invention permet d'obtenir une composition de couleur blanche, insoluble dans l'eau, présentant une clarté définie par un paramètre L^{∗} supérieur à 90 et une opacité supérieure à 80%.

### Utilisations

Selon un dernier aspect, l'invention concerne l'utilisation d'une composition selon l'invention dans des formulations telles que des encres, des peintures, des plastiques, des formulations de produits cosmétiques et des médicaments.

Préférentiellement, la composition selon l'invention représente de 0,1 à 20% en poids par rapport au poids total d'une formulation telle qu'une encre, une peinture, un plastique, une formulation de produit cosmétique et de médicament.

En particulier, l'invention vise l'utilisation d'une telle composition de couleur blanche en tant que substituant du TiO2, dans de telles formulations cosmétiques.

Selon un autre mode de réalisation, l'invention vise l'utilisation de la composition selon l'invention dans des peintures ou encres solvantées.

La composition selon l'invention peut être utilisée notamment dans des peintures choisies parmi la famille des polyuréthanes, acryliques ou époxydes.

La composition selon l'invention peut être également notamment utilisée dans des encres choisies parmi la famille des époxy, acryliques, solvants naphta, nitrocellulosiques, alkydes, polyuréthanes, polyamides, cétoniques ou polyesters.

Avantageusement, la composition selon l'invention permet de remplacer le TiO2 en présentant un paramètre de clarté équivalent à celui du TiO2.

### Exemples

Exemple de composition selon l'invention :

### Exemple 1 :

Une composition selon l'invention (référence 1) a été réalisée selon le protocole suivant :
Référence 1 : La composition selon l'invention (référence 1) comprend :
- de l'oxychlorure de bismuth et du nitrure de bore dans un ratio 0,13 / 1; et
- de l'oxyde de zinc représentant moins de 15% de la masse totale de la composition.

Les mesures du paramètre L^{∗} et d'opacité ont été réalisées et ces mesures ont été comparées à des compositions commerciales visant les mêmes propriétés ainsi qu'au TiO2 utilisée couramment dans les compositions cosmétiques jusqu'à ce jour :
Référence 2 : TiO2 organophile W877 : pigment inorganique comprenant du rutile d'oxyde de titane revêtu d'hydroxyde aluminium, commercialisé par la société Sensient.
Référence 3 : NOVAWHITE^{®} mica/Zn : pigment inorganique comprenant un mélange d'oxyde de zinc et de mica, commercialisé par la société The Innovation Company.
Référence 4 : NOVAWHITE^{®} Zn/Bo : pigment inorganique comprenant un mélange d'oxyde de zinc, d'oxychlorure de bismuth, revêtu d'un mélange d'huile hydrogénée, commercialisé par la société The Innovation Company.
Référence 5 : BN PUHP 1109C : nitrure de bore commercialisé par la société Saint-Gobain

Protocole d'évaluation de L^{∗} et de l'opacité :
1. Préparation de l'échantillon
   - Disperser 20% en massique du pigment à tester dans 80% en massique de résine MARASTAR SR 910 E21
   - Homogénéiser l'échantillon par agitation manuelle, à l'aide d'une spatule, pendant 5 minutes
   - Passer la dispersion obtenue à la tricylindre EXAKT 50i, écartement 1-1
   - Déposer le film sur carte de contraste BYK à l'aide d'un applicateur automatique BYK Byko Drive XL et d'un altère 50
   - Laisser sécher l'échantillon 30 minutes à l'air libre à TA
   - Vérifier que l'épaisseur sèche soit de 35 +/- 5µm à l'aide d'un micromètre (palmer)
2. Mesure de L^{∗} et C sur les échantillons suivant :
   - Le paramètre L^{∗} est déterminé par mesure à l'aide d'un colorimètre Xrite CI62 sur fond blanc et fond noir
   - L'opacité C est déterminée par mesure à l'aide d'un colorimètre Xrite CI62 sur fond blanc et fond noir

**[Tableau 1]**

| **Référence** | **L^{∗}** | **Opacité (%)** |
|---|---|---|
| 1 | 92,21 | 92,04 |
| 2 | 95,39 | 95,82 |
| 3 | 70,75 | 55,53 |
| 4 | 73,39 | 54,67 |
| 5 | 31,09 | 58,07 |

La référence 1, conforme à l'invention présente les propriétés L^{∗} et d'opacité les plus proches de celle de la référence 2 représentant le dioxyde de titane couramment mis en œuvre dans le domaine des compositions cosmétiques. Elle présente également des valeurs de L^{∗} et d'opacité bien plus proche du dioxyde de titane, que les références 3, 4 et 5 proposées comme des alternatives au dioxyde de titane. Enfin comparativement à l'oxychlorure de bismuth qui présente un L^{∗} de 85 et une opacité de 85%, et au nitrure de bore qui présente respectivement un L^{∗} de 90 et une opacité de 86%, la composition de la référence 1 selon l'invention démontre l'effet de synergie dans l'association de ces 2 composés, sur les valeurs de clarté L^{∗} et d'opacité.

### Exemple 2 : Formulation cosmétique :

Une formulation cosmétique de fond de teint comprenant la référence 1 (composition selon l'invention) telle que décrit précédemment a été réalisée selon les éléments du Tableau ci-dessous :

**[Tableau 2]**

| **Nom INCI** | **Noms commerciaux** | **%** |
|---|---|---|
| **Phase huileuse** | | |
| OCTYL PALMITATE | PALMITATE ETHYLE 2 HEXYLE | 15,00 |
| NEOPENTYL GLYCOL DIETHYL-HEXANOATE | DUB DONPG | 6,80 |
| CAPRYLIC/CAPRIC TRIGLYCERIDE | DUB MCT 5545 | 8,00 |
| CYCLOMETHICONE | MIRASIL CM5 | 5,00 |
| PEG-30 DIPOLYHYDROXYSTEARATE | CITHROL DPHS-SO-(MV) | 3,00 |
| CYCLOPENTASILOXANE & DIS-TEARDIMONIUM HECTORITE & SD ALCOHOL 40 | BENTONE GEL VS5V | 2,00 |
| POLYGLYCERYL-3 BEESWAX & TO-COPHEROL & HEXYL CINNAMAL & BENZYL ALCOHOL | CERA BELLINA | 0,25 |

| **Phase aqueuse** | | |
|---|---|---|
| EAU | Eau déminéralisée | 33,00 |
| GLYCERIN | GLYCERINE 4811 | 3,00 |
| BUTYLENE GLYCOL | 1,3 BUTYLENE GLYCOL | 6,00 |
| CAPRYLYL GLYCOL | DERMOSOFT OCTIOL | 0,25 |
| POTASSIUM SORBATE | SORBATE DE POTASSIUM | 0,45 |

| Phase poudreuse | | |
|---|---|---|
| TALC | MICRO ACE P-3 | 7,00 |
| Composition selon l'invention (référence 1 de l'exemple 1) | | 8,00 |
| CI 77492 (IRON OXIDES) & DI-SODIUM STEAROYL GLUTAMATE & ALUMINUM HYDROXIDE | NAI-C339001-10 | 2,25 |

La formulation cosmétique présente de bonne propriété de couvrance, et de tenue, ne colle pas, et est agréable au toucher.

## Revendications

1. Composition de couleur blanche, insoluble dans l'eau, présentant une clarté définie par un paramètre L^{∗} supérieur à 90, une opacité supérieure à 80%, comprenant le mélange d'oxychlorure de Bismuth, de nitrure de bore et d'au moins un autre composé inorganique.

2. Composition selon la revendication 1, **caractérisée en ce que** ledit au moins un autre composé inorganique est choisi parmi : oxyde de fer, oxyde de zinc, oxyde de cérium, carbonate de calcium, phosphate tricalcique, sulfate de calcium, stéarate de zinc, carbonate de magnésium, sulfate de zinc, stéarate de magnésium, sulfate de baryum, sulfure de zinc, poudre d'aluminium, argile calcinée, phosphate tricalcique, kaolin et leur combinaison

3. Composition selon la revendication précédente, **caractérisée en ce que** le au moins un autre composé inorganique est choisi parmi le stéarate de zinc, le sulfate de zinc, l'oxyde de zinc et le sulfure de zinc.

4. Composition selon l'une des revendications précédentes, **caractérisée en ce que** le mélange d'oxychlorure de bismuth et de nitrure de bore représente de 10% à 95%, préférentiellement de 15% à 90% en poids par rapport au poids total de la composition.

5. Composition selon l'une des revendications précédentes, **caractérisée en ce que** le mélange d'oxychlorure de bismuth et de nitrure de bore représente moins de 30%, préférentiellement moins de 25% en poids par rapport au poids total de la composition.

6. Composition selon l'une des revendications précédentes, **caractérisée en ce que** le mélange d'oxychlorure de bismuth et de nitrure de bore représente entre 10 et 50%, préférentiellement entre 10 et 65%, très préférentiellement entre 10 et 80% en poids par rapport au poids total de la composition.

7. Composition selon l'une des précédentes revendications, **caractérisée en ce que** le mélange d'oxychlorure de bismuth et de nitrure de bore présente un ratio compris entre 0,1/1 et 0,7/1, préférentiellement compris entre 0,11/1 et 0,65/1.

8. Composition selon l'une des revendications précédentes, **caractérisée en ce que** la composition présente une prise d'huile inférieure à 55%.

9. Composition selon l'une des précédentes revendications, **caractérisée en ce qu'**elle présente une clarté et/ou une opacité supérieure à celle de chacun des composés constituant la composition.

10. Composition selon l'une des revendications précédentes, **caractérisée en ce que** la composition présente une clarté définie par un paramètre L^{∗} supérieure ou égale à 92 et une opacité supérieure ou égale à 91%.

11. Utilisation d'une composition selon l'une des revendications 1 à 10 dans des encres, des peintures, des plastiques et des formulations de produits cosmétiques, ladite composition représentant de 0,1% à 20% en poids par rapport au poids total de la formulation

12. Procédé de fabrication d'une composition selon l'une des revendications 1 à 10, **caractérisé en ce qu'**il comprend les étapes suivantes :
a) Sélection de chaque composé constituant la composition : oxychlorure de bismuth, nitrure de bore et au moins un autre composé inorganique,
b) Mélange des composés sélectionnés à l'étape a) par ajout successif ; et
c) Récupération d'une composition de couleur blanche présentant une clarté définie par un paramètre L^{∗} supérieur à 90, une opacité supérieure à 80%, et optionnellement une prise en huile inférieure à 55%.

13. Procédé de fabrication d'une composition selon la revendication 12, **caractérisé en ce que** le mélange de l'étape b) est réalisé par dispersion mécanique, à l'aide d'un mélangeur à poudre à une vitesse comprise entre 1 et 20 tr /min, et pendant une durée de 1 à 10 heures.
